# EUROPEAN PATENT APPLICATION

(11) **EP 4 393 471 A1**
(43) Date of publication of application: **03.07.2024**
(21) Application number: 22216780.1
(22) Date of filing: 27.12.2022
(51) Int. Cl.: A61K 8/73, A61K 9/00, A61K 9/06, A61K 31/702

(54) **COSMETIC MANNURONATE, GULURONATE AND GULUMANNURONATE GEL, CREAM OR LOTION AND METHOD FOR ITS PREPARATION**

(71) Applicant: Mirshafiey, Abbas, 14155 Teheran (IR); Lalander, Jacob, 1001 Riga (LV)
(72) Inventor: MIRSHAFIEY, Abbas, 14155 Teheran (IR); LALANDER, Jacob, LV-1001 Riga (LV)
(74) Representative: Maiwald GmbH

(57) **Abstract**

The present invention relates to cosmetic gel, cream or lotion formulations, the formulation comprising β-D-mannuronic acid or α-L-guluronic acid or a mixture of β-D-mannuronic acid and α-L-guluronic acid, oligomers thereof, or cosmetically acceptable salts thereof, wherein the total amount of β-D-mannuronic acid or α-L-guluronic acid or the mixture of β-D-mannuronic acid and α-L-guluronic acid, oligomers thereof, or cosmetically acceptable salts thereof is 0.2 g to 20 g based on 100 g gel, cream or lotion formulation. Additionally, the invention relates to methods for preparing the cosmetic gel, cream or lotion formulations and to the use of said formulations for protecting, reducing or preventing aging and/or for skin care.

## Description

### FIELD OF THE INVENTION

The present invention relates to cosmetic gel, cream or lotion formulations, the formulation comprising β-D-mannuronic acid or α-L-guluronic acid or a mixture of β-D-mannuronic acid and α-L-guluronic acid, oligomers thereof, or cosmetically acceptable salts thereof, wherein the total amount of β-D-mannuronic acid or α-L-guluronic acid or the mixture of β-D-mannuronic acid and α-L-guluronic acid, oligomers thereof, or cosmetically acceptable salts thereof is 0.2 g to 20 g based on 100 g gel, cream or lotion formulation. Additionally, the invention relates to methods for preparing the cosmetic gel, cream or lotion formulations and to the use of said formulations for protecting, reducing or preventing aging and/or for skin care.

### BACKGROUND OF THE INVENTION

Mannuronic acid and guluronic acid are natural uronic acids which are isolated and purified from various forms of alginates, which are polymers used as a thickener, binder, gelling agent or lubricant. The source of alginate can be brown sea-weeds, e.g., *Phaeophyceae* and kelp, and/or bacteria belonging to the genera *Pseudomonas* and *Azotobacter.* The properties of the alginate vary, depending on which species it comes from. Alginates are mainly available in the form of sodium, calcium and ammonium salts. The difference in their monomer structures (mannuronic and guluronic acids) as well as the distribution, proportion and the length of these monomer blocks determine the chemical and physical properties of alginate polymers and oligomers.

Mannuronic and guluronic acids are epimers of each other. Therefore, many physicochemical and biological properties of these two molecules, e.g., molecular formula (C₆H₁₀O₇), molecular weight (194.14 g/mol), boiling point (553.4±50.0 °C at 760 mmHg), and density (1.7±0.1 g/cm³), are the same or highly similar.

Therapeutic properties of mannuronic acid in various animal models were first reported in 2004. The results of these studies revealed the therapeutic efficacy and tolerability of mannuronic acid in *in vitro* and *in vivo* experiments. The potent therapeutic efficacy of mannuronic acid was reported by Mirshafiey et al. 2005 (Mirshafiey A, Cuzzocrea S, Rehm BHA, H. Matsuo H. "M2000: a revolution in pharmacology", Med Sci Monit. 2005. 11(8):153-163). Later studies were carried out to address safety profile, the underlying cellular, molecular and immunological mechanisms and its therapeutic effects at the level of human clinical trials. The results of various studies and clinical trials confirmed that mannuronic acid is very safe and provides for a wide spectrum of therapeutic effects in a variety of diseases, such as rheumatoid arthritis, ankylosing spondylitis, breast cancer, multiple sclerosis, and myelodisplastic syndrome (Fattahi MJ, Jamshidi AR, Mahmoudi M, et al. "Evaluation of the efficacy and safety of β-d-mannuronic acid in patients with ankylosing spondylitis", "A 12-week randomized, placebo-controlled, phase I/II clinical trial", International Immunopharmacology. 2018; 54:112-117; Ahmadi H, Jamshidi AR, Gharibdoost F, et al, "A phase I/II randomized, controlled, clinical trial for assessment of the efficacy and safety of β-D-mannuronic acid in rheumatoid arthritis patients". Inflammopharmacology. 2018; 26(3):737-745; Rezaieyazdi Z, Farooqi A, Soleymani-Salehabadi H, et al. "International multicenter randomized, placebo-controlled phase III clinical trial of β-D-mannuronic acid in rheumatoid arthritis patients". Inflammopharmacology. 2019; 27(5):911-921; Kashefi S, Omranipour R, Mahmoodzadeh H, Ahmadi H, Mirshafiey A. "Clinical improvement of diabetes mellitus type 1 by β-D-mannuronic acid (M2000) in a breast cancer patient - as a case report", Clin Diabetol. 2019; 8(4):227-229; Najafi S, Moghadam NB, Saadat P, et al. "A controlled, randomized phase II clinical trial for efficacy and safety evaluation of mannuronic acid in secondary progressive form of multiple sclerosis", Int J Neurosci. 2022; 132(4):403-412; Ghaderi A, Nodehi SRS, Bakhtiari T, et al, "Mannuronic Acid in Low-Risk and Intermediate-1-Risk Myelodysplastic Syndromes". J Clin Pharmacol. 2020; 60(7):879-888).

The systematic pharmacology and medical investigations on guluronic acid were started in 2013 by Mirshafiey et al, and the results were published in 2015 ("New therapeutic approach by G2013 in experimental model of multiple sclerosis" Afraei S, Azizi G, Zargar SJ, Sedaghat R, Mirshafiey A., in; Acta Neurol Belg. 2015 Sep;115(3):259-66). Furthermore, the safety and pharmacotoxicology characteristics of guluronic acid as well as its clinical efficacy on inflammatory diseases have been studied.

These studies showed that not only share these two uronic acids many physicochemical properties, but also have the same or similar biological and medical properties.

Oxidation is a chemical reaction that can produce free radicals or chain reactions that may damage the cells of an organism. Antiaging substances are, e.g., antioxidants that inhibit and/or reduce oxidation. For example, antioxidants such as thiols or vitamins A, C, or E may catch or eliminate free radicals and thereby prevent cell damage.

There is always a need in the art for novel products with antioxidant efficacy.

### SUMMARY OF THE INVENTION

Surprisingly, it has been found that β-D-mannuronic acid or α-L-guluronic acid, an oligomer thereof, or a cosmetically acceptable salt thereof or mixtures of β-D-mannuronic acid and α-L-guluronic acid, oligomers thereof, or cosmetically acceptable salts thereof, preferably mannuronate or guluronate or gulumannuronate, can be formulated in cosmetic gels, creams or lotions, preferably for topical use, in particular as antioxidant agents with antiaging property and/or as a skin care against aging phenomena.

Therefore, in a first aspect, the present invention relates to a cosmetic gel, cream or lotion formulation, the formulation comprising
(i) β-D-mannuronic acid, an oligomer thereof, or a cosmetically acceptable salt thereof, or
(ii) α-L-guluronic acid, an oligomer thereof, or a cosmetically acceptable salt thereof, or
(iii) a mixture of β-D-mannuronic acid, an oligomer thereof, or a cosmetically acceptable salt thereof and α-L-guluronic acid, an oligomer thereof, or a cosmetically acceptable salt thereof, preferably gulumannuronate,
wherein the total amount of β-D-mannuronic acid or α-L-guluronic acid, or their mixture, oligomers thereof or cosmetically acceptable salts thereof ranges from 0.2 g to 20 g based on 100 g gel, cream or lotion formulation.

In various embodiments,
(i) the β-D-mannuronic acid, an oligomer thereof, or a cosmetically acceptable salt thereof is β-D-mannuronate, preferably selected from the group consisting of sodium β-D-mannuronate, potassium β-D-mannuronate, magnesium β-D-mannuronate, calcium β-D-mannuronate or ammonium β-D-mannuronate and combinations thereof, more preferably sodium β-D-mannuronate; and/or
(ii) the α-L-guluronic acid, an oligomer thereof, or a cosmetically acceptable salt thereof is α-L-guluronate, preferably selected from the group consisting of sodium α-L-guluronate, potassium α-L-guluronate, magnesium α-L-guluronate, calcium α-L-guluronate or ammonium α-L-guluronate and combinations thereof, more preferably sodium α-L-guluronate.

In various embodiments where a mixture of β-D-mannuronic acid and α-L-guluronic acid, the respective oligomers thereof, or the respective cosmetically acceptable salts thereof is used, the active agent may be gulumannuronate, preferably
wherein β-D-mannuronic acid and α-L-guluronic acid, oligomers thereof, or cosmetically acceptable salts thereof are mixed and preferably selected from the group consisting of sodium, or potassium, or magnesium, or calcium, or ammonium β-D-mannuronate and/or α-L-guluronate and combinations thereof, most preferably sodium β-D-mannuronate and/or sodium α-L-guluronate, or
wherein the mixture of β-D-mannuronic acid and α-L-guluronic acid, oligomers thereof, or cosmetically acceptable salts thereof, preferably gulumannuronate, is alginate hydrolysate (powder).

In various embodiments, the total amount of
(i) β-D-mannuronic acid, an oligomer thereof, or a cosmetically acceptable salt thereof, or
(ii) α-L-guluronic acid, an oligomer thereof, or a cosmetically acceptable salt thereof, or
(iii) the mixture of β-D-mannuronic acid and α-L-guluronic acid, oligomers thereof, or cosmetically acceptable salts thereof,
in the cosmetic gel, cream or lotion formulation is 0.5 g to 15 g/100 g gel, cream or lotion, preferably 1 g to 10 g/100 g gel, cream or lotion, more preferably 2 g to 8 g/100 g gel, cream or lotion, more preferably 2.5 g to 6 g/100 g gel, cream or lotion, most preferably 3 g to 5 g/100 g gel, cream or lotion, e.g. 3 g/100 g gel, cream or lotion.

In various embodiments, the cosmetic gel, cream or lotion formulation can further comprise at least one further active (cosmetic) agent and/or at least one further ingredient as described herein.

Preferably, the cosmetic gel, cream or lotion formulation is an antiaging cosmetic gel, cream or lotion, preferably an antioxidant cosmetic gel, cream or lotion, or a skin care cosmetic gel, cream or lotion.

In a second aspect, the present invention relates to a method for preparing a cosmetic gel, cream or lotion formulation as described herein, preferably an antiaging or skin care cosmetic gel, cream or lotion.

In various embodiments, the method for preparing a cosmetic gel, cream or lotion formulation according to the invention is a method is for preparing a cosmetic cream formulation according to the invention, wherein the method comprises the steps of
(i) providing a (first) composition comprising at least one cream-forming substance and, optionally, at least one further active (cosmetic) agent and/or at least one further ingredient, preferably at least one emulsifier, at least one plasticizer and/or at least one preservative;
(ii) providing a (second) composition:
   (a) comprising β-D-mannuronic acid, an oligomer thereof, or a cosmetically acceptable salt thereof, preferably β-D-mannuronate, in a total amount of 0.2 g to 20 g, preferably 3 g, based on 100 g cream formulation; or
   (b) comprising α-L-guluronic acid, an oligomer thereof, or a cosmetically acceptable salt thereof, preferably α-L-guluronate, in a total amount of 0.2 g to 20 g, preferably 3 g, based on 100 g cream formulation; or
   (c) comprising a mixture of β-D-mannuronic acid and α-L-guluronic acid, oligomers thereof, or cosmetically acceptable salts thereof, preferably gulumannuronate, in a total amount of 0.2 g to 20 g, preferably 3 g, based on 100 g cream formulation;
(iii) optionally adjusting the pH of the second solution to pH 1 to 5, preferably 2 to 4, more preferably 3;
(iv) mixing the solutions of i) and ii); and
(v) optionally adjusting the pH to 4 to 8, more preferably 5 to 7, most preferably 5.5 to 6.5.

In a third aspect, the invention relates to the use of the cosmetic gel, cream or lotion formulation according to the invention for
(i) protecting, reducing or preventing aging, preferably as an antioxidant cosmetic gel, cream or lotion; and/or
(ii) skin care, preferably for skin protection against cell damage and aging process.

These and other aspects, embodiments, features, and advantages of the invention become apparent to the person skilled in the art in the following detailed description and claims. Each feature from one aspect of the invention can be used in any other aspect of the invention. Furthermore, the examples contained herein are intended to describe and illustrate the invention, but do not restrict it. In particular, the invention is not limited to these examples.

### DETAILED DESCRIPTION

"At least one", as used herein, means one or more, i.e. 1, 2, 3, 4, 5, 6, 7, 8, 9 or more of the referenced species. Similarly, "one or more", as used herein, relates to at least one and comprises 1, 2, 3, 4, 5, 6, 7, 8, 9 or more. In connection with a given species, the term does not relate to the total number of molecules, but rather to the type of species. "At least one preservative", for example, thus means that one type of preservative or two or more different types of preservatives may be present. In connection with amounts, the term relates to the total amount of the referenced species. In case of preservatives, for example, this means that the given amount is the total amount of all preservatives in the composition/formulation.

As used herein, unless otherwise stated, the singular forms "a", "an", and "the" include plural reference. Thus, for example, a reference to "an oligomer thereof' also includes a plurality of oligomer molecules or oligomer types, e.g. a mixture or combination of various oligomer types. Furthermore, unless otherwise stated, the use of plural forms, e.g. "oligomers thereof' or "cosmetically acceptable salts thereof", also includes singular reference such as one type of oligomer or one type of salt.

Numeric values specified without decimal places herein refer to the full value specified with one decimal place, i.e. for example, 99 % means 99.0 %, unless otherwise defined.

The term "about", in connection with a numerical value, refers to a variance of ±10 %, preferably ±5 %, with respect to the given numerical value.

The term "essentially free" within the context of this invention is to be interpreted as the respective compound is contained in the formulation in an amount of less than 5 wt.-%, 4 wt.-%, 3 wt.-%, 2 wt.-%, 1.5 wt.-%, 1 wt.-%, 0.75 wt.-%, 0.5 wt.-%, 0.25 wt.-%, 0.1 wt.-%, 0.01 wt.-%, or 0.001 wt.-% based on the total weight of the formulation, wherein the amounts are respectively more preferred in descending order. For example, 4 wt.-% is more preferred than 5 wt.-% and 3 wt.-% is more preferred than 4 wt.-%.

All percentages given herein in relation to the formulations relate to weight-% (wt.-%) relative to the total weight of the respective formulations, if not explicitly stated otherwise. Numeric ranges specified in the format "from x to y" include the specified values. If multiple preferred numeric ranges are specified in this format, it is understood that all ranges created by combining the different endpoints are also included.

In the following, the term "gel, cream or lotion formulation" is used to describe the cosmetic gel, cream or lotion formulation, preferably the antiaging cosmetic gel, cream or lotion formulation. The terms "gel(s)" and "gel formulation(s)", "cream(s)" and "cream formulation(s)" and "lotion(s)" or "lotion formulation(s)" are intended to encompass all viscous formulation types typically used in the field and include pastes and ointments.

In the context of the present invention, a "cream formulation" may be a two-phase, (semi-solid) dosage form that contains an aqueous phase in addition to a lipid phase (emulsion). In various embodiments, the cream can be a hydrophilic cream or a lipophilic cream.

In the context of the present invention, a "gel formulation" may be a (semi-solid) finely dispersed system with at least two liquid phases or at least one liquid and at least one solid phase, the continuous phase preferably comprising a gel-forming substance. Suitable gel formulations are, for example, water-based gels and alcohol-based gels, without being limited thereto, wherein water-based gel formulations are especially preferred in the present invention.

In the context of the present invention, a "lotion formulation" may be a liquid aqueous or aqueousalcoholic preparation, preferably with suspended or emulsified active agents and excipients. Preferably, a lotion is an oil-in-water emulsion and the water content in lotions is higher than in creams or ointments.

The viscosity is an important physical property of (topical) gel, cream or lotion formulations, which can also affect the rate of drug release. Viscosity can be measured in various ways, including vibrating viscometers, capillary viscometers, rotational rheometry, microfluidic rheometers and non-contact rheology. If not defined otherwise, all viscosities mentioned herein are determined by rotational rheometry. Preferably, the formulation according to the invention has a viscosity in the range of 1000 cPs to 40000 cPs, preferably in the range of 2000 cPs to 30000 cPs, more preferably in the range of 3000 cPs to 20000 cPs, most preferably in the range of 3500 cPs to 10000 cPs determined in a laboratory digital Brookfield viscometer (rotational viscometer) at 25°C. In various embodiments, the formulation is subjected to a steady-state-flow method (0.1-100 s⁻¹) to characterize the flow property and to obtain viscosity values at low (2.0 s⁻¹), medium (20.0 s⁻¹), and high (75.0 s-¹) shear rates at 25°C. In preferred embodiments, the shear rate (shear viscosity value) for gel is 75.0 - 85.0 s-¹, for cream is 60.0 - 70.0 s-¹ and for lotion is 80.0 - 90.0 s-¹, in particular the formulation according to the invention has a viscosity in the range of 1000 cPs to 40000 cPs, preferably in the range of 2000 cPs to 30000 cPs, more preferably in the range of 3000 cPs to 20000 cPs, most preferably in the range of 3500 cPs to 10000 cPs, determined in a laboratory digital Brookfield viscometer at 25°C and at a shear rate for gel of 75.0 s⁻¹ - 85.0 s⁻¹, for cream of 60.0 s⁻¹ - 70.0 s⁻¹ or for lotion of 80.0 s⁻¹ - 90.0 s⁻¹.

Beta-D-mannuronic acid has the following formula (I):

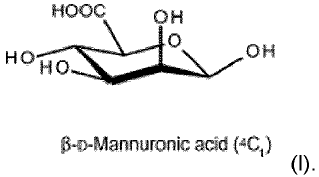

Alpha-L-guluronic acid has the following formula (II):

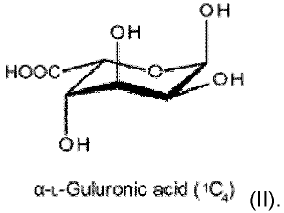

As mannuronic acid and guluronic acid are epimers, they share many physicochemical properties as well as biological and medicinal properties, for example their antiaging effect, e.g. as antioxidants. Therefore, in the present invention, all concepts disclosed for β-D-mannuronic acid, an oligomer thereof, or a cosmetically acceptable salt thereof are similarly applicable to α-L-guluronic acid, an oligomer thereof, or a cosmetically acceptable salt thereof, or to mixtures of both, and *vice versa.*

According to the invention, β-D-mannuronic acid or α-L-guluronic acid or their mixtures can be comprised in the cosmetic gel, cream or lotion formulation in form of cosmetically acceptable salts. Preferably, these cosmetically acceptable salts are mannuronate, guluronate, or a mixture thereof, more preferably β-D-mannuronate, or α-L-guluronate, or a mixture thereof referred to as "gulumannuronate". The counterion may be selected from sodium, potassium, magnesium, calcium, ammonium and other cosmetically acceptable cationic counterions.

Thus, in various embodiments, the cosmetic gel, cream or lotion formulation can be:
(i) a mannuronate gel, cream or lotion formulation; or
(ii) a guluronate gel, cream or lotion formulation; or
(iii) a gulumannuronate gel, cream or lotion formulation.

In various embodiments, the cosmetic gel, cream or lotion formulation comprising β-D-mannuronic acid, an oligomer thereof, or a cosmetically acceptable salt thereof, is a mannuronate gel, cream or lotion formulation. Specifically, the β-D-mannuronic acid, an oligomer thereof, or a cosmetically acceptable salt thereof can be, in various embodiments, β-D-mannuronate, more preferably selected from the group consisting of sodium β-D-mannuronate, potassium β-D-mannuronate, magnesium β-D-mannuronate, calcium β-D-mannuronate, ammonium β-D-mannuronate and combinations thereof, with sodium β-D-mannuronate being especially preferred.

In various embodiments, β-D-mannuronic acid, preferably β-D-mannuronate, can also be added to the cosmetic gel, cream or lotion formulation in the form of a precursor substance selected from oligomers of β-D-mannuronic acid or β-D-mannuronate, preferably (homo)oligomers of β-D-mannuronate, in particular sodium oligomannuronate, potassium oligomannuronate, magnesium oligomannuronate, calcium oligomannuronate, ammonium oligomannuronate and combinations thereof, especially sodium oligomannuronate.

In various embodiments, β-D-mannuronic acid, an oligomer thereof, or a cosmetically acceptable salt thereof, preferably β-D-mannuronate, is selected from the group consisting of sodium β-D-mannuronate, potassium β-D-mannuronate, magnesium β-D-mannuronate, calcium β-D-mannuronate, ammonium β-D-mannuronate, sodium oligomannuronate, potassium oligomannuronate, magnesium oligomannuronate, calcium oligomannuronate, ammonium oligomannuronate and combinations thereof, preferably sodium β-D-mannuronate and/or sodium oligomannuronate.

In various embodiments, the cosmetic gel, cream or lotion formulation comprising α-L-guluronic acid, an oligomer thereof, or a cosmetically acceptable salt thereof is a guluronate gel, cream or lotion formulation. Specifically, the α-L-guluronic acid, an oligomer thereof, or a cosmetically acceptable salt thereof can be, in various embodiments, α-L-guluronate, preferably selected from the group consisting of sodium α-L-guluronate, potassium α-L-guluronate, magnesium α-L-guluronate, calcium α-L-guluronate, ammonium α-L-guluronate and combinations thereof, with sodium α-L-guluronate being especially preferred.

In various embodiments, α-L-guluronic acid, preferably α-L-guluronate, can also be added to the cosmetic gel, cream or lotion formulation in the form of a precursor substance selected from oligomers of α-L-guluronic acid or α-L-guluronate, preferably (homo)oligomers of α-L-guluronate, in particular sodium oligoguluronate, potassium oligoguluronate, magnesium oligoguluronate, calcium oligoguluronate, ammonium oligoguluronate and combinations thereof, especially sodium oligoguluronate.

In various embodiments, α-L-guluronic acid, an oligomer thereof, or a cosmetically acceptable salt thereof, preferably α-L-guluronate, is selected from the group consisting of sodium α-L-guluronate, potassium α-L-guluronate, magnesium α-L-guluronate, calcium α-L-guluronate, ammonium α-L-guluronate, sodium oligoguluronate, potassium oligoguluronate, magnesium oligoguluronate, calcium oligoguluronate, ammonium oligoguluronate and combinations thereof, preferably sodium α-L-guluronate and/or sodium oligoguluronate.

In various embodiments, the cosmetic gel, cream or lotion formulation comprising a mixture of β-D-mannuronic acid and α-L-guluronic acid, oligomers thereof, or cosmetically acceptable salts thereof, is a gulumannuronate gel, cream or lotion formulation. In particular, the mixture of β-D-mannuronic acid and α-L-guluronic acid, oligomers thereof, or cosmetically acceptable salts thereof can be a mixture of β-D-mannuronate and α-L-guluronate, preferably selected from the group consisting of sodium β-D-mannuronate, potassium β-D-mannuronate, magnesium β-D-mannuronate, calcium β-D-mannuronate, ammonium β-D-mannuronate or combinations thereof and sodium α-L-guluronate, potassium α-L-guluronate, magnesium α-L-guluronate, calcium α-L-guluronate, ammonium α-L-guluronate or combinations thereof, with a mixture of sodium β-D-mannuronate and sodium α-L-guluronate being especially preferred.

In various embodiments, the mixture of β-D-mannuronic acid and α-L-guluronic acid, preferably gulumannuronate, can also be added to the cosmetic gel, cream or lotion formulation in the form of precursor substances selected from oligomers of β-D-mannuronic acid/β-D-mannuronate and/or α-L-guluronic acid/α-L-guturonate, preferably (homo)oligomers of β-D-mannuronic acid/β-D-mannuronate and/or α-L-guluronic acid/α-L**-**guluronate, in particular sodium oligomannuronate, potassium oligomannuronate, magnesium oligomannuronate, calcium oligomannuronate, ammonium oligomannuronate, sodium oligoguluronate, potassium oligoguluronate, magnesium oligoguluronate, calcium oligoguluronate, ammonium oligoguluronate and combinations thereof, especially sodium oligomannuronate and/or sodium oligoguluronate.

In various embodiments, β-D-mannuronic acid and/or α-L-guluronic acid, oligomers thereof, or cosmetically acceptable salts thereof, preferably gulumannuronate, is selected from the group consisting of sodium β-D-mannuronate, potassium β-D-mannuronate, magnesium β-D-mannuronate, calcium β-D-mannuronate, ammonium β-D-mannuronate, sodium oligomannuronate, potassium oligomannuronate, magnesium oligomannuronate, calcium oligomannuronate, ammonium oligomannuronate, sodium α-L-guluronate, potassium α-L-guluronate, magnesium α-L-guluronate, calcium α-L-guluronate, ammonium α-L-guluronate, sodium oligoguluronate, potassium oligoguluronate, magnesium oligoguluronate, calcium oligoguluronate, ammonium oligoguluronate and combinations thereof, preferably sodium β-D-mannuronate, sodium oligomannuronate, sodium α-L-guluronate or sodium oligoguluronate and combinations thereof.

In particular, β-D-mannuronate oligomers and/or α-L-guluronate oligomers comprise 2 to 16 monomeric units, preferably 3 to 6 monomeric units, e.g., 3 or 4 or 5 or 6 monomeric units.

In various embodiments, the mixture of β-D-mannuronic acid and α-L-guluronic acid, oligomers thereof, or cosmetically acceptable salts thereof, preferably gulumannuronate, can be synthesized from sodium alginate and can be used in the cosmetic gel, cream or lotion formulation in the form of alginate hydrolysate, preferably alginate hydrolysate powder, which is also referred to as gulumannuronate. In particular, alginate hydrolysate (gulumannuronate) is the separated precipitate of sodium alginate comprising mannuronic acid/mannuronate and guluronic acid/guluronate. Therefore, in various embodiments, the cosmetic gel, cream or lotion formulation comprises alginate hydrolysate (gulumannuronate) in concentrations as described herein, preferably 0.2 g to 20 g/100 g gel,cream or lotion, more preferably 0.5 g to 15 g/100g gel, cream or lotion, more preferably 1 g to 10 g/100 g gel, cream or lotion, more preferably 2 g to 8 g/100 g gel, cream or lotion, more preferably 2.5 g to 6 g/100 g gel, cream or lotion, most preferably 3 g to 5 g/100g gel, cream or lotion.

It is preferred that the mixture of β-D-mannuronic acid and α-L-guluronic acid, oligomers thereof, or cosmetically acceptable salts thereof, preferably gulumannuronate, may be
(1) a mixture of the single components β-D-mannuronic acid, oligomers thereof, or cosmetically acceptable salts thereof, in particular β-D-mannuronate (powder) and α-L-guluronic acid, oligomers thereof, or cosmetically acceptable salts thereof, in particular α-L-guluronate (powder); or
(2) alginate hydrolysate (powder).

In various embodiments, the total amount of
(i) β-D-mannuronic acid, an oligomer thereof or a cosmetically acceptable salt thereof, or
(ii) α-L-guluronic acid, an oligomer thereof, or a cosmetically acceptable salt thereof, or
(iii) the mixture of β-D-mannuronic acid or α-L-guluronic acid, oligomers thereof, or cosmetically acceptable salts thereof,
in the cosmetic gel, cream or lotion formulation is 0.5 g to 15 g/100g gel, cream or lotion, more preferably 1 g to 10 g/100 g gel, cream or lotion, e.g., 1 g, 2 g, 3 g, 4 g, or 5 g based on 100 g gel, cream or lotion, more preferably 2 g to 8 g/100 g gel, cream or lotion, more preferably 2.5 g to 6 g/100 g gel, cream or lotion, more preferably 3 g to 5 g/100g gel, cream or lotion.

In one embodiment, the total amount of β-D-mannuronic acid, an oligomer thereof, or a cosmetically acceptable salt thereof in the cosmetic gel, cream or lotion formulation, preferably in the mannuronate gel, cream or lotion formulation, is 0.5 g to 15 g/100g gel, cream or lotion, preferably 1 g to 10 g/100 g gel, cream or lotion, more preferably 2 g to 8 g/100 g gel, cream or lotion, more preferably 2.5 g to 6 g/100 g gel, cream or lotion, most preferably 3 g to 5 g/100g gel, cream or lotion, e.g. 3 g/100 g gel, cream or lotion.

In another embodiment, the total amount of α-L-guluronic acid, an oligomer thereof, or a cosmetically acceptable salt thereof in the cosmetic gel, cream or lotion formulation, preferably in the guluronate gel, cream or lotion formulation, is 0.5 g to 15 g/100g gel, cream or lotion, preferably 1 g to 10 g/100 g gel, cream or lotion, more preferably 2 g to 8 g/100 g gel, cream or lotion, more preferably 2.5 g to 6 g/100 g gel, cream or lotion, most preferably 3 g to 5 g/100g gel, cream or lotion, e.g. 3 g/100 g gel, cream or lotion.

In another embodiment, the total amount of the mixture of β-D-mannuronic acid and α-L-guluronic acid, oligomers thereof, or cosmetically acceptable salts thereof in the cosmetic gel, cream or lotion formulation, preferably in the gulumannuronate gel, cream or lotion formulation, is 0.5 g to 15 g/100g gel, cream or lotion, preferably 1 g to 10 g/100 g gel, cream or lotion, more preferably 2 g to 8 g/100 g gel, cream or lotion, more preferably 2.5 g to 6 g/100 g gel, cream or lotion, most preferably 3 g to 5 g/100g gel, cream or lotion, e.g. 3 g/100 g gel, cream or lotion.

In various embodiments, the mixture of β-D-mannuronic acid and α-L-guluronic acid, oligomers thereof, or cosmetically acceptable salts thereof, preferably (in) the gulumannuronate gel, cream or lotion formulation, comprises or consists of
0.1 g to 19.9 g of β-D-mannuronic acid, an oligomer thereof or a cosmetically acceptable salt thereof, and
0.1 g to 19.9 g of α-L-guluronic acid, an oligomer thereof, or a cosmetically acceptable salt thereof,
with the proviso that the total amount of β-D-mannuronic acid, an oligomer thereof, or a cosmetically acceptable salt thereof and α-L-guluronic acid, an oligomer thereof, or a cosmetically acceptable salt thereof is at least 0.2 g and at most 20 g based on 100 g gel, cream or lotion formulation; preferably the total amount of β-D-mannuronic acid, an oligomer thereof, or a cosmetically acceptable salt thereof is 0.5 g to 15 g, preferably 0.8 to 10 g, more preferably 1 g to 8 g, more preferably 1.2 g to 5 g, more preferably 1.5 g to 5 g, most preferably 1.5 g, based on 100 g gel, cream or lotion formulation, and the total amount of α-L-guluronic acid, an oligomer thereof, or a cosmetically acceptable salt thereof is 0.5 g to 15 g, preferably 0.8 g to 10 g, more preferably 1 g to 8 g, more preferably 1.2 g to 5 g, more preferably 1.5 g to 5 g, most preferably 1.5 g, based on 100 g gel, cream or lotion formulation. In various embodiments, the total of β-D-mannuronic acid and α-L-guluronic acid, oligomers thereof or cosmetically acceptable salts thereof could be: 3 g/100 g gel, cream or lotion, 5.01 g/100 g gel, cream or lotion, 10 g/100 g gel, cream or lotion, 15 g/100 g gel, cream or lotion or 20 g/100 g gel, cream or lotion. In one specific embodiment, the mixture comprises or consists of 1.5 g β-D-mannuronic acid, an oligomer thereof or a cosmetically acceptable salt thereof and 1.5 g α-L-guluronic acid, an oligomer thereof, or a cosmetically acceptable salt thereof based on 100 g gel, cream or lotion formulation. In various embodiments, the weight ratio of β-D-mannuronic acid, an oligomer thereof, or a cosmetically acceptable salt thereof to α-L-guluronic acid, an oligomer thereof, or a cosmetically acceptable salt thereof is in the range of from 100:1 to 1:100, such as 10:1 to 1:10 or 5:1 to 1:5, preferably 3:1 to 1:3, 2:1 to 1:2 or about 1.5:1 to 1:1.5, most preferably about 1:1.

The concentration ranges mentioned above for the different cosmetic gel, cream or lotion formulations mean that the gel or cream formulation according to the invention comprises 0.2 to 20 wt.-% of
(i) β-D-mannuronic acid, an oligomer thereof, or a cosmetically acceptable salt thereof, or
(ii) α-L-guluronic acid, an oligomer thereof, or a cosmetically acceptable salt thereof, or
(iii) the mixture of β-D-mannuronic acid, an oligomer thereof, or a cosmetically acceptable salt thereof and α-L-guluronic acid, an oligomer thereof, or a cosmetically acceptable salt thereof,
preferably 0.5 to 15 wt.-%, such as 3 wt.-%, 5 wt.-%, 10 wt.-%, 15 wt.-%, more preferably 1 to 10 wt.-%, more preferably 2 to 8 wt.-%, more preferably 2.5 to 6 wt.-%, more preferably 3 to 5 wt.-%, based on the total weight of the gel, cream or lotion formulation.

In various embodiments, the cosmetic gel, cream or lotion formulation according to the invention can comprise β-D-mannuronic acid or α-L-guluronic acid, oligomers thereof, or cosmetically acceptable salts thereof, or their mixture, preferably β-D-mannuronate or α-L-guluronate or their mixture, together with other, preferably chemically pure, supplement agents and/or active agents for cosmetic use.

In various embodiments, the cosmetic gel, cream or lotion formulation may comprise at least one further active (cosmetic) agent.

In various embodiments, without being limited thereto, the at least one further active (cosmetic) agent is selected from antioxidant agents, anti-inflammatory agents, and agents with anti-sensitivity properties or combinations thereof.

Suitable antioxidant agents are, for example, without being limited thereto, thiols or vitamins A, C, or E.

A suitable anti-inflammatory agent according to the invention is, for example, an agent that reduces or eliminates inflammation or inflammatory reaction in the body and/or at the skin.

Agents with anti-sensitivity properties which can be added to the cosmetic gel, cream or lotion formulation according to the invention are all anti-allergy or anti-sensitive (desensitizing) agents safe for cosmetic use in humans. Examples are, without being limited thereto, antihistamines and diphenhydramine HCl.

Therefore, in various embodiments, the cosmetic gel, cream or lotion formulation according to the invention is an antiaging cosmetic gel, cream or lotion formulation, and/or an antioxidant cosmetic gel, cream or lotion formulation and/or a gel, cream or lotion formulation with skin care properties.

In various embodiments, the cosmetic gel, cream or lotion formulation further comprises caffeic acid or a salt thereof, for example in powder form, preferably in an amount of 0.001 wt.-% to 1 wt.-%, more preferably 0.01 wt.-% to 0.5 wt.-%, more preferably 0.05 wt.-% to 0.3 wt.-%, e.g., 0.1 wt.-% to 0.3 wt.%, based on the total weight of the cosmetic gel, cream or lotion formulation.

Caffeic acid (3,4-dihydroxycinnamic acid, with the molecular formula C₉H₈O₄) may be found in many plants and foods. For example, coffee is the primary source of caffeic acid in the human diet. However, it can also be found in other food sources such as apples, pears, artichoke and berries. In various embodiments, caffeic acid may have many effects in the body including antioxidant, anti-inflammatory, neuroprotective and photoprotective effects. Caffeic acid is commercially available, for example from Sigma Aldrich, or can be isolated from coffee or other food sources.

Therefore, in various embodiments, the cosmetic gel, cream or lotion formulation comprises
(i) β-D-mannuronic acid, an oligomer thereof, or a cosmetically acceptable salt thereof, or
(ii) α-L-guluronic acid, an oligomer thereof, or a cosmetically acceptable salt thereof, or
(iii) a mixture of β-D-mannuronic acid, an oligomer thereof, or a cosmetically acceptable salt thereof and α-L-guluronic acid, an oligomer thereof, or a cosmetically acceptable salt thereof, preferably gulumannuronate,

wherein the total amount of β-D-mannuronic acid or α-L-guluronic acid, or their mixture, oligomers thereof, or cosmetically acceptable salts thereof is 0.2 wt.-% to 20 wt.-%, based on the total weight of the cosmetic gel, cream or lotion formulation;
in combination with caffeic acid (powder), preferably in amounts of 0.001 wt.-% to 1 wt.-%, more preferably 0.01 wt.-% to 0.5 wt.-%, more preferably 0.05 wt.-% to 0.3 wt.-%, e.g., 0.1 wt.-% to 0.3 wt.%, based on the total weight of the cosmetic gel, cream or lotion formulation. It is preferred that such a formulation may be used as an antiaging cosmetic gel, cream or lotion formulation and/or antioxidant cosmetic gel, cream or lotion formulation and/or gel, cream or lotion formulation with skin care properties.

Preferably, the cosmetic gel, cream or lotion formulation is for topical use or a topical cosmetic gel, cream or lotion formulation.

In various embodiments, the cosmetic gel, cream or lotion formulation according to the invention is for topical use in a method of protecting, reducing or preventing aging, preferably as an antioxidant cosmetic gel, cream or lotion and/or skin care, e.g., for skin protection against cell damage, aging process and/or aging pathogenesis.

In various embodiments, the cosmetic gel, cream or lotion formulation may contain further ingredients such as (one or more) auxiliaries (carriers, skin protectants, disinfectants, preservatives, cream-forming substances, gel-forming substances, lotion-forming substances, emulsifiers, etc.) known to those skilled in the art and conventionally used in such formulations.

For example, the following auxiliaries may be used in the formulations of the invention, without being limited thereto: particulate carriers (e.g., talc, zinc oxide, starch, starch derivatives, diatomaceous earth); gel-forming substances (e.g., gelatin, tragacanth, cellulose, cellulose derivatives, alginates, polyacrylic acid); cream-forming substances (e.g., lanolin, beeswax, olive oil, arachis oil, liquid paraffin); humectants (e.g., urea, glycerin, propylene glycol), pressure-sensitive polymers (e.g., polyacrylates, and adhesive resins); ointment bases (e.g., petroleum jelly, fats, cellulose derivatives, polyacrylic acid, alginates); emulsifiers (e.g., sodium lauryl sulphate "SLS", stearic acid, lanolin, lecithin, sorbitan esters, monoglycerides); preservatives (e.g., alcohols, glycols, benzoates and their derivatives, e.g., benzalkonium chloride); antioxidants (e.g., butylated hydroxyanisole, vitamin E, ascorbic acid and its derivatives); thickeners (e.g., hydroxypropylmethylcellulose); pH adjusting agents; binders (e.g., polyvinylpyrrolidone, starch, hydroxypropylmethylcellulose, polyethylene glycols); fillers (e.g., microcrystalline cellulose, sorbitol); colorants; flavorings; sweeteners (e.g., sorbitol, aspartame); solvents (e.g., water, ethanol, ethanol-water mixtures); solubilizers (e.g., glycerol, propylene glycol); skin penetration enhancers (e.g., alcohols, terpenes, propylene glycol); plasticizers (e.g., sorbitol, glycerin, phthalic acid esters); wetting agents (e.g., sodium lauryl sulfate, polysorbates); synthetic and natural oils (e.g. medium-chain triglycerides, paraffin, synthetic and/or natural wax, almond sweet oil); propellants for aerosol or foam sprays (e.g. norflurane, cryoflurane, dichlorofluoromethane, trichlorofluoromethane, propane, butane, isobutane, nitrogen).

In various embodiments, the at least one further ingredient is selected from preservatives, alkaline agents, colorants, fragrances, gel-forming substances, cream-forming substances, lotion-forming substances, emulsifiers, thickeners and combinations thereof.

In various embodiments, the cosmetic gel, cream or lotion formulation comprises at least one preservative, preferably selected from the group of anti-bacterial agents, anti-fungal agents, alcohols, glycols, benzoates and/or their derivatives, essential oil or combinations thereof. Preferred preservatives are, e.g., anti-bacterial agents, anti-fungal agents, propylene glycols, butylene glycols, pentylene glycols, ethoxydiglycol, phenoxyethanol, benzyl benzoate, methylisothiazolinone, zinc pyrithione, benzalkonium chloride, sodium benzoate, alcohols, essential oils or derivatives or combinations thereof, without being limited thereto.

It is possible that the cosmetic gel, cream or lotion formulation is free of or essentially free of parabens.

In various embodiments, the cosmetic gel, cream or lotion formulation is free of or essentially free of anti-bacterial agents and/or anti-fungal agents.

In various embodiments, the cosmetic gel, cream or lotion formulation is free of or essentially free of parabens and anti-bacterial agents and anti-fungal agents.

It is preferred that the cosmetic gel, cream or lotion formulation is a cream formulation.

Preferably, the cosmetic cream formulation according to the invention comprises at least one cream-forming substance and/or at least one emulsifier and/or at least one thickener.

Suitable cream-forming substances are, for example, lanolin, beeswax, olive oil, coconut oil, avocado oil, arachis oil, argan oil, and/or petrolatum (petroleum jelly), liquid paraffin, essential oils, mineral oils, glycerine, zinc oxide, butyl stearate and diglycol laurate, or combinations thereof, without being limited thereto.

Preferably, suitable gel-forming, cream-forming and lotion-forming substances are selected from cosmetic grade components, in particular from pharmaceutical grade components.

In various embodiments, sodium carboxymethylcellulose, hydroxyethylcellulose, carbomer (polyacrylates/polyacrylic acid; e.g. in powder form) and xanthan gum can advantageously be used as the gel-forming substance.

Especially, the at least one gel-forming substance is selected from carbomers ((homo)polymers of acrylic acid and salts thereof), cellulose, alginates, or derivatives or combinations thereof, preferably in a cosmetical grade quality, in particular in a pharmaceutical grade quality.

In particular carbomers (acrylic acid polymers) are used as a suitable basis for many commercial gels, which absorb water readily upon contact and forming a gel.

Carbomers are commercially available, e.g., under the trade names Carbomer 104, Carbomer 934, Carbomer 934 P, Carbomer 940, Carbomer 941, Carbomer 974P, Carbomer 980, Carbomer 1342, Carbomer sodium salt, etc., wherein the numbers refer to the molecule size and the adhesive force.

In preferred embodiments, Carbomer 934 (P) is comprised in a cosmetic gel formulation according to the invention.

The gel-forming substance, preferably a carbomer, is preferably present in an amount of between about 0.8 wt.-% and about 6.0 wt.-%, more preferably between about 1.5 wt.-% and about 4 wt.-%, more preferably between about 2.0 wt.-% and about 3.0 wt.-%, e.g. 2.5 wt.-%, based on the total weight of the gel formulation.

Suitable lotion-forming substances are, for example, selected from the group of mineral and vegetable oils, silicone-based oils, plant or animal derived waxes, gums, cellulose and its derivatives, and fatty materials, without being limited thereto.

In a cosmetic cream formulation, ingredients are preferably dissolved or dispersed in either a water-in-oil emulsion (W/O emulsion) or an oil-in-water emulsion (O/W emulsion). In various embodiments, they are present in a suitable vehicle, or base, which may be optimised for a particular site of the body or type of skin condition. A cosmetic cream may basically be a mixture of oil and water (O/W). Preferably, a (topical) cosmetic cream formulation has a higher content of oily substance than a gel, but a lower content of oily ingredient than an ointment.

If the cosmetic gel, cream or lotion formulation is a cream formulation, it preferably comprises at least one emulsifier.

Suitable emulsifiers can be anionic, cationic, neutral, amphoteric or complex emulsifiers or combinations thereof, and/or synthetic or natural emulsifiers or combinations thereof. In preferred embodiments, the at least one emulsifier is a synthetic and/or natural emulsifier, preferably selected from the group consisting of lecithin, lanolin, sorbitan ester, cetyl alcohol, cetearyl alcohol, stearic acid, glyceryl (mono)stearate, mono-/diglyceride, sodium lauryl sulphate (SLS) and benzalkonium chloride, polyoxyl castor oil, carnauba wax, candelilla wax, beeswax, or combinations thereof.

Preferred natural emulsifier are, e.g., lanolin, lecithin, carnauba wax, candelilla wax, beeswax or combinations thereof.

In various embodiments, the cosmetic gel, cream or lotion formulation may comprise at least one alkaline agent, for example in the form of a solution, typically to adjust the pH value of the formulation.

Preferably, the cosmetic gel, cream or lotion formulation is administered topically, optionally to a subject in need thereof.

In preferred embodiments, the subject is a mammalian, in particular a human.

In preferred embodiments, the cosmetic gel, cream or lotion formulation according to the invention is for topical use in a method of
(i) protecting, reducing or preventing aging, preferably as an antioxidant cosmetic gel, cream or lotion; and/or
(ii) skin care, preferably for skin protection against cell damage and/or aging process and/or antiaging pathogenesis.

In a further aspect, the present invention relates to a method for preparing a cosmetic gel, cream or lotion formulation according to the invention.

According to the invention, the resulting cosmetic gel, cream or lotion formulation obtained by the method according to the invention comprises
(i) β-D-mannuronic acid, an oligomer thereof, or a cosmetically acceptable salt thereof, preferably β-D-mannuronate, or
(ii) α-L-guluronic acid, an oligomer thereof, or a cosmetically acceptable salt thereof, preferably α-L-guluronate, or
(iii) the mixture of β-D-mannuronic acid, an oligomer thereof, or a cosmetically acceptable salt thereof, preferably β-D-mannuronate, and α-L-guluronic acid, an oligomer thereof, or a cosmetically acceptable salt thereof, preferably α-L-guluronate,
in a total amount of 0.2 g to 20 g based on 100 g gel, cream or lotion formulation, preferably in a total amount of 0.5 g to 15 g/100 g gel, cream or lotion, more preferably in a total amount of 1 g to 10 g/100g gel, cream or lotion such as 1g, 2 g, 3 g, 4 g, or 5 g based on 100g gel, cream or lotion, more preferably 2 g to 8 g/100 g gel, cream or lotion, most preferably 3 g to 5 g/100 g gel, cream or lotion.

This means that the cosmetic gel, cream or lotion formulation according to the invention comprises 0.2 to 20 wt.-% of
(i) β-D-mannuronic acid, an oligomer thereof, or a cosmetically acceptable salt thereof, or
(ii) α-L-guluronic acid, an oligomer thereof, or a cosmetically acceptable salt thereof, or
(iii) the mixture of β-D-mannuronic acid, an oligomer thereof, or a cosmetically acceptable salt thereof and α-L-guluronic acid, an oligomer thereof, or a cosmetically acceptable salt thereof,
preferably 0.5 to 15 wt.-%, more preferably 1 to 10 wt.-% such as 1 wt.-%, 2 wt.-%, 3 wt.-%, 4 wt-% or 5 wt.-%, more preferably 2 to 8 wt.-%, more preferably 2.5 to 6 wt.-% most preferably 3 to 5 wt.-%, based on the total weight of the gel, cream or lotion formulation, preferably 100 g gel, cream or lotion formulation (final formulation).

In various embodiments, β-D-mannuronic acid, an oligomer thereof, or a cosmetically acceptable salt thereof, preferably β-D-mannuronate, and/or α-L-guluronic acid, an oligomer thereof, or a cosmetically acceptable salt thereof, preferably α-L-guluronate, is added in the method according to the invention in powder form.

In embodiments in which the resulting cosmetic gel, cream or lotion formulation is the cosmetic gulumannuronate gel, cream or lotion formulation comprising a mixture of β-D-mannuronic acid and α-L-guluronic acid, oligomers thereof, or cosmetically acceptable salts thereof, preferably β-D-mannuronate and α-L-guluronate, the gel, cream or lotion formulation preferably comprises or consists of
0.1 g to 19.9 g of β-D-mannuronic acid, an oligomer thereof, or a cosmetically acceptable salt thereof, and
0.1 g to 19.9 g of α-L-guluronic acid, an oligomer thereof, or a cosmetically acceptable salt thereof, with the proviso that the total amount of β-D-mannuronic acid, an oligomer thereof, or a cosmetically acceptable salt thereof and α-L-guluronic acid, an oligomer thereof, or a cosmetically acceptable salt thereof is at least 0.2 g and at most 20 g, based on 100 g gel, cream or lotion formulation; preferably the total amount of β-D-mannuronic acid, an oligomer thereof, or a cosmetically acceptable salt thereof is 0.5 g to 15 g, more preferably 0.8 g to 10 g, more preferably 1 g to 8 g, more preferably 1.2 g to 6 g, more preferably 1.5 g to 5 g, most preferably 1.5 g, based on 100 g gel, cream or lotion formulation, and the amount of α-L-guluronic acid, an oligomer thereof, or a cosmetically acceptable salt thereof is 0.5 g to 15 g, preferably 0.8 g to 10 g, more preferably 1 g to 8 g, more preferably 1.2 g to 6 g, more preferably 1.5 g to 5 g, most preferably 1.5 g, based on 100 g gel, cream or lotion formulation.

In various embodiments, alginate hydrolysate (powder) can be used (as the mixture of β-D-mannuronic acid, an oligomer thereof, or a cosmetically acceptable salt thereof and α-L-guluronic acid, an oligomer thereof, or a cosmetically acceptable salt thereof) for the cosmetic gulumannuronate gel, cream or lotion preparation. Preferably, alginate hydrolysate (powder) is used in total amounts of 0.2 g to 20 g/100 g gel, cream or lotion formulation, more preferably 0.5 g to 10 g/100 g gel, cream or lotion formulation, most preferably 3 g/100g gel, cream or lotion formulation.

In various embodiments, β-D-mannuronic acid, an oligomer thereof, or a cosmetically acceptable salt thereof, preferably β-D-mannuronate, is selected from the group consisting of sodium β-D-mannuronate, potassium β-D-mannuronate, magnesium β-D-mannuronate, calcium β-D-mannuronate, ammonium β-D-mannuronate, and combinations thereof, and/or sodium oligomannuronate, potassium oligomannuronate, magnesium oligomannuronate, calcium oligomannuronate, ammonium oligomannuronate and combinations thereof, with sodium β-D-mannuronate and/or sodium oligomannuronate being especially preferred in the cosmetic mannuronate gel, cream or lotion preparation.

In various embodiments, α-L-guluronic acid, an oligomer thereof, or a cosmetically acceptable salt thereof, preferably α-L-guluronate, is selected from the group consisting of sodium α-L-guluronate, potassium α-L-guluronate, magnesium α-L-guluronate, calcium α-L-guluronate, ammonium α-L-guluronate, and combinations thereof, and/or sodium oligoguluronate, potassium oligoguluronate, magnesium oligoguluronate, calcium oligoguluronate, ammonium oligoguluronate and combinations thereof, with sodium α-L-guluronate and/or sodium oligoguluronate being especially preferred in the cosmetic guluronate gel, cream or lotion preparation.

In various embodiments, the mixture of β-D-mannuronic acid and α-L-guluronic acid, oligomers thereof, or cosmetically acceptable salts thereof, preferably β-D-mannuronate and α-L-guluronate, are selected from the group consisting of sodium or potassium or magnesium or calcium or ammonium β-D-mannuronate and sodium or potassium or magnesium or calcium or ammonium α-L-guluronate and combinations thereof, and/or their oligomers, preferably their homooligomers consisting of sodium or potassium or magnesium or calcium or ammonium oligomannuronate and sodium or potassium or magnesium or calcium or ammonium oligoguluronate and combinations thereof, with sodium β-D-mannuronate, sodium α-L-guluronate, sodium oligomannuronate or sodium oligoguluronate, and combination thereof being preferred in the cosmetic gulumannuronate gel, cream or lotion preparation.

In particular, oligomannuronate or oligoguluronate comprise 2 to 16 β-D-mannuronic acid or α-L-guluronic acid monomers, preferably 3 to 6 β-D-mannuronic acid or α-L-guluronic acid monomers, e.g., 3 or 4 or 5 or 6 β-D-mannuronic acid or α-L-guluronic acid monomers.

In preferred embodiments, the cosmetic gel, cream or lotion formulation obtained by the method according to the invention may comprise at least one further active (cosmetic) agent, preferably selected from antioxidant agents, anti-inflammatory agents and/or agents with anti-sensitivity properties as described above.

Therefore, in various embodiments, the method according to the invention is a method for preparing an antiaging or skin care cosmetic gel, cream or lotion formulation, e.g. an antioxidant cosmetic gel, cream or lotion formulation.

It is further possible that the cosmetic gel, cream or lotion formulation obtained by the method according to the invention comprises at least one further ingredient such as one or more auxiliaries as described herein.

Preferably, the method is for preparing a cosmetic cream formulation according to the invention, wherein the method comprises the steps of
(i) providing a (first) composition comprising at least one cream-forming substance and, optionally, at least one further active (cosmetic) agent and/or at least one further ingredient, preferably at least one emulsifier, at least one plasticizer and/or at least one preservative;
(ii) providing a (second) composition:
   (a) comprising β-D-mannuronic acid, an oligomer thereof, or a cosmetically acceptable salt thereof, preferably β-D-mannuronate, in a total amount of 0.2 g to 20 g, preferably 3 g, based on 100 g cream formulation; or
   (b) comprising α-L-guluronic acid, an oligomer thereof, or a cosmetically acceptable salt thereof, preferably α-L-guluronate, in a total amount of 0.2 g to 20 g, preferably 3 g, based on 100 g cream formulation; or
   (c) comprising a mixture of β-D-mannuronic acid and α-L-guluronic acid, oligomers thereof, or cosmetically acceptable salts thereof, preferably gulumannuronate, in a total amount of 0.2 g to 20 g, preferably 3 g, based on 100 g cream formulation;
(iii) optionally adjusting the pH of the second solution to pH 1 to 5, preferably 2 to 4, more preferably 3;
(iv) mixing the solutions of i) and ii); and
(v) optionally adjusting the pH to 4 to 8, more preferably 5 to 7, most preferably 5.5 to 6.5.

In various embodiments, in a (topical) cream formulation, ingredients are dissolved or dispersed in either a water-in-oil (W/O) emulsion or an oil-in-water (O/W) emulsion.

Preferably, in step i), a semi-solid composition or emulsion, preferably a water-in-oil (W/O) emulsion or an oil-in-water (O/W) emulsion, is prepared. The composition may comprise at least one cream forming substance, and, optionally, at least one further active (cosmetic) agent and/or at least one further ingredient, preferably at least one emulsifier, at least one plasticizer and/or at least one preservative. In various embodiments, the at least one cream-forming substance and, optionally, the at least one emulsifier, the at least one plasticizer and/or the at least one preservative is/are dissolved/mixed in/with water, preferably deionized water.

Further ingredients include, for example, at least one thickener as described herein and/or at least one (organic) solvent such as polyethylene glycol (PEG), without being limited thereto.

In various preferred embodiments, the (second) composition of step ii) is an aqueous composition. It is further preferred that the β-D-mannuronic acid, an oligomer thereof, or a cosmetically acceptable salt thereof, preferably β-D-mannuronate for mannuronate cosmetic cream preparation, or α-L-guluronic acid, an oligomer thereof, or a cosmetically acceptable salt thereof, preferably α-L-guluronate for guluronate cosmetic cream preparation, or the mixture of β-D-mannuronic acid and α-L-guluronic acid, oligomers thereof, or cosmetically acceptable salts thereof, preferably β-D-mannuronate and α-L-guluronate, in particular gulumannuronate, for gulumannuronate cosmetic cream preparation is dissolved in water, preferably deionized water, to obtain the (second) composition. The concentrations are preferably as described above for the general method for preparing a cosmetic gel, cream or lotion formulation, such that the concentrations in the final formulation are as described herein. In various embodiments, alginate hydrolysate (powder) can be used as gulumannuronate (as the mixture of β-D-mannuronic acid, an oligomer thereof, or a cosmetically acceptable salt thereof and α-L-guluronic acid, an oligomer thereof, or a cosmetically acceptable salt thereof) for gulumannuronate cosmetic cream preparation and is dissolved in water, preferably deionized water, to obtain the (second) solution. Preferably, alginate hydrolysate (powder) is added in amounts such that a concentration of 0.2 g to 20 g/100 g cream formulation, more preferably 1 g to 10 g/100 g cream formulation, most preferably 3 g to 5 g/100g cream formulation is obtained.

In various embodiments, the second composition may further comprise at least one further active (cosmetic) agent and/or at least one further ingredient.

After preparation of the compositions of step i) and ii), the composition of step ii) is mixed with the composition of step i).

In various embodiments, before and/or after mixing, an alkaline agent solution is added to at least one of the compositions, preferably to the (second) composition of step ii) or to the cream formulation after mixing to adjust the pH. If the cream is a water-based cream (O/W base), the alkaline agent solution is preferably a NaOH solution.

In preferred embodiments, an alkaline agent solution, preferably a NaOH solution, is added to the dissolved mannuronic acid, an oligomer thereof, or a cosmetically acceptable salt thereof, or guluronic acid, an oligomer thereof, or a cosmetically acceptable salt thereof, or the mixture of mannuronic acid and guluronic acid, oligomers thereof, or cosmetically acceptable salts thereof of step ii) to adjust the pH, preferably to a pH of 1 to 5, more preferably 2 to 4, e.g., to a pH of 3.

Preferably, after mixing of the compositions of steps i) and ii), the pH is adjusted to 4 to 8, more preferably 5 to 7, most preferably 5.5 to 6.5.

In various embodiments, the viscosity of the produced cream is adjusted in a range of 1000 cPs to 40000 cPs, preferably in the range of 2000 cPs to 30000 cPs, more preferably in the range of 3000 cPs to 20000 cPs, most preferably in the range of 3500 cPs to 10000 cPs, determined in a laboratory digital Brookfield viscometer (rotational viscometer) at 25°C, preferably at a shear rate of 60.0 s⁻¹ - 70.0 s⁻¹.

In preferred embodiments, the composition of step i) or the composition of step ii) may comprise at least one further active (cosmetic) agent, preferably selected from antioxidant agents, anti-inflammatory agents and/or agents with anti-sensitivity properties as described above.

In various embodiments, the method according to the invention is a method for preparing an antiaging cosmetic cream formulation, an antioxidant cosmetic cream formulation, a skin care cosmetic cream formulation, an anti-inflammatory cosmetic cream formulation and/or a cosmetic cream formulation with anti-sensitivity properties. Preferably, the cosmetic cream formulation is administered topically.

It is further possible that the (first) composition of step i) and/or the (second) composition of step ii) comprise(s) at least one further ingredient such as one or more auxiliaries as described herein.

The further active (cosmetic) agents and/or the further ingredient (auxiliary) may also be comprised in a further composition, e.g., in a third or fourth composition that may be added in later steps.

Preferably, in further compositions, the pH can be adjusted, preferably as described above, before mixing with the compositions of step i) and/or ii).

In various embodiments, further compositions, if present, are mixed with the composition of step i) and/or with the composition of step ii) or with the mixture of both. Afterwards, preferably the pH is adjusted to pH 4 to 8, more preferably 5 to 7, most preferably 5.5 to 6.5.

Additional water can be added to the resulting composition to obtain a cream formulation according to the invention. This step is an optional step to adjust the final concentrations of the ingredients of the cosmetic cream formulations and/or to adapt the consistence and/or viscosity of the cosmetic cream formulation, for example.

In various embodiments, the method comprises the steps of
(i) providing a (first) composition comprising at least one cream-forming substance and, optionally, at least one further active (cosmetic) agent and/or at least one further ingredient, e.g., at least one emulsifier, at least one plasticizer and/or at least one preservative;
(ii) providing a (second) composition comprising
   (a) β-D-mannuronic acid, an oligomer thereof, or a cosmetically acceptable salt thereof, preferably β-D-mannuronate, in a total amount of 0.2 g to 20 g, preferably 0.5 g to 15 g, more preferably 1 g to 10 g, more preferably 2 g to 8 g, most preferably 3 g to 5 g, based on 100 g cream formulation; or
   (b) α-L-guluronic acid, an oligomer thereof, or a cosmetically acceptable salt thereof, preferably α-L-guluronate, in a total amount of 0.2 g to 20 g, preferably 0.5 g to 15 g, more preferably 1 g to 10 g, more preferably 2 g to 8 g, most preferably 3 g to 5 gbased on 100 g cream formulation; or
   (c) a mixture of β-D-mannuronic acid and α-L-guluronic acid, oligomers thereof, or cosmetically acceptable salts thereof, preferably gulumannuronate, in a total amount of 0.2 g to 20 g, preferably 0.5 to 15 g, more preferably 1 g to 10 g, more preferably 2 g to 8 g, most preferably 3 g to 5 g, based on 100 g cream formulation;
   and optionally at least one further active (cosmetic) agent and/or at least one further ingredient;
(iii) optionally providing at least one further composition comprising at least one further active (cosmetic) agent and/or at least one further ingredient;
(iv) optionally adjusting the pH of the second composition to pH 1 to 5, preferably 2 to 4, more preferably 3;
(v) mixing the compositions of step i) and step ii), and, optionally, step iii);
(vi) optionally adjusting the pH of the resulting composition to 4 to 8, more preferably 5 to 7, most preferably 5.5 to 6.5;
(vii) optionally adjusting the viscosity of the resulting composition to 1000 cPs to 40000 cPs, preferably 2000 cPs to 30000 cPs, more preferably 3000 cPs to 20000 cPs, most preferably 3500 cPs to 10000 cPs determined in a laboratory digital Brookfield viscometer (rotational viscometer) at 25°C, preferably at a shear rate of 60.0 s⁻¹ - 70.0 s⁻¹;
(viii) optionally adding water to the resulting composition to obtain a cosmetic cream formulation, preferably a cosmetic cream formulation as described herein.

A further aspect of the invention is the use of the cosmetic gel, cream or lotion formulation according to the invention for
(i) protecting, reducing or preventing aging, preferably as an antioxidant cosmetic gel, cream or lotion; and/or
(ii) skin care, preferably for skin protection against cell damage, aging process and/or aging pathogenesis.

Preferably, β-D-mannuronic acid and/or α-L-guluronic acid, oligomers thereof, or cosmetically acceptable salts thereof are active (cosmetic) agents acting as antioxidants. In particular, they may catch or eliminate free radicals in the cells and prevent chain reactions that could otherwise damage (skin) cells.

In various embodiments, the resulting cosmetic gel, cream or lotion formulation can be sterilised. Suitable methods for sterilising the formulation include, by way of example, heat/steam treatment, radiation and aseptic production.

The cosmetic gel, cream or lotion formulation according to the invention can be filled or packaged in any suitable packaging, e.g., tubes, films or other packages. These may be made of any suitable material, such as aluminium-based tubes or low melting point polyolefin packages, such as polyethylene packages.

All embodiments and examples described herein for the cosmetic gel, cream or lotion formulation according to the invention also apply to the method for preparing said gel, cream or lotion formulations and to the use of said formulations and *vice versa.*

Other embodiments are within the following non-limiting examples.

### Examples

### 1. Method of preparation of mannuronic acid, guluronic acid and gulumannuronate powders from sodium alginate.

Mannuronic acid, guluronic acid and gulumannuronate were synthesized according to the GMP criteria of the WHO for substance production.

To synthesize "β-D-mannuronic acid" and "α-L-guluronic acid" and "gulumannuronate", the alginic acid sodium salt (sodium alginate) was used. In a first step, sodium alginate (100 g) was dissolved gently in 1500 ml H₂SO₄ 20% at 0 °C. The solution was thoroughly stirred at room temperature and was then heated to 85 °C until its color changed from a light cream color to light brown. The hydrolysate was allowed to cool to room temperature and the precipitate separated by centrifugation (3700 g). This precipitate can be used as gulumannuronate (alginate hydrolysate). The precipitate was re-dissolved by neutralization using 1 M Na₂CO₃ solution. This solution was then adjusted to pH 2.85 using 0.5 M HCl and the precipitate again separated by centrifugation (3700 g). The precipitate was collected and washed once with distilled water. This precipitate (α-L-guturonic acid) was spread and dried in petri dishes to yield α-L-guluronic acid in powder form. Said powder can be used in guluronate gel, cream or lotion preparation and/or in the mixture of β-D-mannuronic acid and α-L-guluronic acid for gulumannuronate gel, cream or lotion preparation. The remaining supernatant of the L-guluronic acid precipitate was collected and adjusted to pH 1.0 using 0.5 M HCI. Following centrifugation (3700 g), the precipitate was collected and washed once with distilled water. The obtained precipitate (β-D-mannuronic acid) was spread and dried in petri dishes to yield β-D-mannuronic acid in powder form. This may be used in mannuronate gel, cream or lotion preparation and/or in the mixture of β-D-mannuronic acid with α-L-guluronic acid for gulumannuronate gel, cream or lotion preparation. The characteristics of α-L-guluronic acid and β-D-mannuronic acid and their purity were validated by Fourier Transform Infrared (FT-IR) spectroscopy and Carbon-13 Nuclear Magnetic Resonance (13C-NMR) spectroscopy.

The provided powders (mannuronic acid) and (guluronic acid) were stored at room temperature (22 - 26 °C) in a dry place under sterile conditions in order to prepare the cosmetic gel, cream or lotion.

### 2. Method of preparation of mannuronate, guluronate and gulumannuronate topical cosmetic cream

I): In the first stage, 5.0% arachis oil, 7.0% liquid paraffin, 6.0% polyoxyl castor oils (emulsifier), 2.0% acid stable glyceryl monostearate (emulsifier), and 15% cetyl stearyl 2-ethyl hexanoate (plasticizer or emoillent) are mixed and heated at 80°C and then 0.3% methyl hydroxybenzoate and 0.2% propyl hydroxybenzoate (preservatives) along with 3.0% propylene glycol are added. This composition is referred to as (Composition 1).
II): In a separate glass beaker:
   IIa): for mannuronate cream preparation, 3 g mannuronic powder is added to 20 ml deionized water and then adjusted to pH 3 by adding NaOH 30% to completely dissolve the mannuronate (Composition 2a);
   IIb): for guluronate cream preparation, 3 g guluronic powder is added to 20 ml deionized water and then adjusted to pH 3 by adding NaOH 30% to completely dissolve the guluronate (Composition 2b);
   IIc): for gulumannuronate cream preparation, 1.5 g mannuronic powder and 1.5 g guluronic powder are added to 20 ml deionized water and adjusted to pH 3 by adding NaOH 30% to dissolve the powders (Composition 2c). An alternative way for gulumannuronate cream preparation is to use the first precipitate during the sodium alginate hydrolysis process. For this purpose, 3 g gulumannuronte (alginate hydrolysate) powder is added to 20 ml deionized water and is dissolved by adding NaOH 30% until pH 3 (Composition 2c).
III): After adding (Composition 2) to (Composition 1) and obtaining a clear solution, the pH is slowly adjusted to 5.5 - 6 (Composition 3).
IV): Deionized water is added to (Composition 3) up to a total (final) weight of 100 g.
V): The synthesized mannuronate cosmetic cream (using composition 2a) and guluronate cosmetic cream (using composition 2b) and gulumannuronate cosmetic cream (using composition 2c) can be packaged under sterile conditions and then stored.

## Claims

1. A cosmetic gel, cream or lotion formulation, the formulation comprising
(i) β-D-mannuronic acid, an oligomer thereof, or a cosmetically acceptable salt thereof, or
(ii) α-L-guluronic acid, an oligomer thereof, or a cosmetically acceptable salt thereof, or
(iii) a mixture of β-D-mannuronic acid, an oligomer thereof, or a cosmetically acceptable salt thereof and α-L-guluronic acid, an oligomer thereof, or a cosmetically acceptable salt thereof, preferably gulumannuronate,
wherein the total amount of β-D-mannuronic acid or α-L-guluronic acid, or their mixture, oligomers thereof, or cosmetically acceptable salts thereof is 0.2 g to 20 g based on 100 g gel, cream or lotion formulation.

2. The cosmetic gel, cream or lotion formulation according to claim 1, wherein the gel, cream or lotion formulation has a viscosity of 1000 cPs to 40000 cPs, preferably in the range of 2000 cPs to 30000 cPs, more preferably in the range of 3000 cPs to 20000 cPs, most preferably in the range of 3500 cPs to 10000 cPs, determined in a laboratory digital Brookfield viscometer (rotational viscometer) at 25°C, preferably at a shear rate for gel of 75.0 s⁻¹ - 85.0 s-¹, for cream of 60.0 s⁻¹ - 70.0 s⁻¹ or for lotion of 80.0 s⁻¹ - 90.0 s⁻¹.

3. The cosmetic gel, cream or lotion formulation according to claim 1 or 2, wherein
(i) the β-D-mannuronic acid, an oligomer thereof, or a cosmetically acceptable salt thereof is β-D-mannuronate, preferably selected from the group consisting of sodium β-D-mannuronate, potassium β-D-mannuronate, magnesium β-D-mannuronate, calcium β-D-mannuronate, or ammonium β-D-mannuronate and combinations thereof, more preferably sodium β-D-mannuronate; or
(ii) the α-L-guluronic acid, an oligomer thereof, or a cosmetically acceptable salt thereof is α-L-guluronate, preferably selected from the group consisting of sodium α-L-guluronate, potassium α-L-guluronate, magnesium α-L-guluronate, calcium α-L-guluronate, or ammonium α-L-guluronate and combinations thereof, more preferably sodium α-L-guluronate; or
(iii) the mixture of β-D-mannuronic acid and α-L-guluronic acid, oligomers thereof, or cosmetically acceptable salts thereof, preferably gulumannuronate, is selected from the group consisting of sodium, or potassium, or magnesium, or calcium, or ammonium β-D-mannuronate and α-L-guluronate and combinations thereof, preferably sodium β-D-mannuronate and/or sodium α-L-guluronate; or
(iv) the mixture of β-D-mannuronic acid and α-L-guluronic acid, oligomers thereof, or cosmetically acceptable salts thereof, preferably gulumannuronate, is alginate hydrolysate (powder).

4. The cosmetic gel, cream or lotion formulation according to any one of claims 1 to 3, wherein β-D-mannuronic acid and/or α-L-guluronic acid, oligomers thereof, or cosmetically acceptable salts thereof are selected from oligomers of β-D-mannuronate and/or α-L-guluronate.

5. The cosmetic gel, cream or lotion formulation according to claim 4, wherein the oligomer of β-D-mannuronate and/or α-L-guluronate
(i) is the homooligomer of β-D-mannuronate and/or the homooligomer of α-L-guluronate;
(ii) is selected from the group consisting of sodium, or potassium, or magnesium, or calcium, or ammonium oligomannuronate and oligoguluronate and combinations thereof, preferably sodium oligomannuronate and/or sodium oligoguluronate; and/or
(iii) comprises or consists of 2 to 16 β-D-mannuronic acid monomers and/or 2 to 16 α-L-guluronic acid monomers.

6. The cosmetic gel, cream or lotion formulation according to any one of claims 1 to 5, wherein the total amount of
(i) β-D-mannuronic acid, an oligomer thereof, or a cosmetically acceptable salt thereof, or
(ii) α-L-guluronic acid, an oligomer thereof, or a cosmetically acceptable salt thereof, or
(iii) the mixture of β-D-mannuronic acid and α-L-guluronic acid, oligomers thereof, or cosmetically acceptable salts thereof,
in the cosmetic gel, cream or lotion formulation is 0.5 g to 15 g/100 g gel, cream or lotion, preferably 1 g to 10 g/100 g gel, cream or lotion, more preferably 2 g to 8 g/100 g gel, cream or lotion, most preferably 3 g to 5 g/100 g gel, cream or lotion.

7. The cosmetic gel, cream or lotion formulation according to claim 6, wherein the mixture of β-D-mannuronic acid and α-L-guluronic acid, oligomers thereof, or cosmetically acceptable salts thereof, preferably the gulumannuronate formulation, comprises or consists of 0.1 g to 19.9 g of β-D-mannuronic acid, an oligomer thereof, or a cosmetically acceptable salt thereof, and 0.1 g to 19.9 g of α-L-guluronic acid, an oligomer thereof, or a cosmetically acceptable salt thereof, with the proviso that the total amount of β-D-mannuronic acid, an oligomer thereof, or a cosmetically acceptable salt thereof and α-L-guluronic acid, an oligomer thereof, or a cosmetically acceptable salt thereof is at least 0.2 g and at most 20 g, based on 100 g gel, cream or lotion formulation; preferably
the total amount of β-D-mannuronic acid, an oligomer thereof, or a cosmetically acceptable salt thereof is 0.5 g to 15 g, preferably 0.8 g to 10 g, more preferably 1 g to 8 g, more preferably 1.5 g to 5 g, most preferably 1.5 g, based on 100 g gel, cream or lotion formulation, and
the total amount of α-L-guluronic acid, an oligomer thereof, or a cosmetically acceptable salt thereof is 0.5 g to 15 g, preferably 0.8 g to 10 g, more preferably 1 g to 8 g, more preferably 1.5 g to 5 g, most preferably 1.5 g, based on 100 g gel, cream or lotion formulation.

8. The cosmetic gel, cream or lotion formulation according to any one of claims 1 to 7, wherein the gel, cream or lotion formulation further comprises at least one further ingredient selected from preservatives, antioxidants, alkalis, dyes, perfumes, fragrances, cream-forming substances, gel-forming substances, lotion-forming substances and emulsifiers and combinations thereof.

9. The cosmetic gel, cream or lotion formulation according to claim 8, wherein the at least one preservative is selected from the group of anti-bacterial agents, anti-fungal agents, alcohols, glycols, benzoates and/or their derivatives, essential oil or combinations thereof.

10. The cosmetic gel, cream or lotion formulation according to any one of claims 1 to 9, wherein the cosmetic gel, cream or lotion formulation is free of or essentially free of parabens and/or anti-bacterial agents and/or anti-fungal agents.

11. The cosmetic gel, cream or lotion formulation according to any one of claims 8 to 10, wherein
(i) the at least one cream-forming substance is lanolin, beeswax, olive oil, coconut oil, avocado oil, arachis oil, argan oil, and/or petrolatum (petroleum jelly), liquid paraffin, essential oils, mineral oils, glycerine, zinc oxide, butyl stearate and diglycol laurate, or combinations thereof; or
(ii) the at least one gel-forming substance is polyacrylic acid (carbomer), cellulose, alginate or their derivatives or combinations thereof, preferably a (homo)polymer of acrylic acid (carbomer); or
(iii) the at least one lotion-forming substance is selected from the group of mineral and vegetable oils, silicone-based oils, plant or animal derived waxes, gums, cellulose and its derivatives and fatty materials.

12. The cosmetic gel, cream or lotion formulation according to any one of claims 8 to 11, wherein the at least one emulsifier is a synthetic or natural emulsifier, preferably selected from the group consisting of lecithins, lanolin, sorbitan ester, cetyl alcohol, cetearyl alcohol, stearic acid, glyceryl (mono)stearate, mono-/diglyceride, sodium lauryl sulphate, benzalkonium chloride, polyoxyl castor oil, carnauba wax, candelilla wax, beeswax or a combination thereof.

13. The cosmetic gel, cream or lotion formulation according to any one of claims 1 to 12, wherein the formulation is an antiaging cosmetic gel, cream or lotion, preferably an antioxidant cosmetic gel, cream or lotion, or a skin care cosmetic gel, cream or lotion.

14. A method for preparing a cosmetic gel, cream or lotion formulation according to any one of claims 1 to 13, wherein the method is preferably for preparing a cosmetic cream formulation, wherein the method comprises the steps of
(i) providing a (first) composition comprising at least one cream-forming substance and, optionally, at least one further active (cosmetic) agent and/or at least one further ingredient, preferably at least one emulsifier, at least one plasticizer and/or at least one preservative;
(ii) providing a (second) composition:
(a) comprising β-D-mannuronic acid, an oligomer thereof, or a cosmetically acceptable salt thereof, preferably β-D-mannuronate, in a total amount of 0.2 g to 20 g, preferably 3 g, based on 100 g cream formulation; or
(b) comprising α-L-guiuronic acid, an oligomer thereof, or a cosmetically acceptable salt thereof, preferably α-L-guluronate, in a total amount of 0.2 g to 20 g, preferably 3 g, based on 100 g cream formulation; or
(c) comprising a mixture of β-D-mannuronic acid and α-L-guluronic acid, oligomers thereof, or cosmetically acceptable salts thereof, preferably gulumannuronate, in a total amount of 0.2 g to 20 g, preferably 3 g, based on 100 g cream formulation;
(iii) optionally adjusting the pH of the second solution to pH 1 to 5, preferably 2 to 4, more preferably 3;
(iv) mixing the solutions of i) and ii); and
(v) optionally adjusting the pH to 4 to 8, more preferably 5 to 7, most preferably 5.5 to 6.5.

15. Use of the cosmetic gel, cream or lotion formulation according to any one of claims 1 to 13 for
(i) protecting, reducing or preventing aging, preferably as an antioxidant cosmetic gel, cream or lotion; and/or
(ii) skin care, preferably for skin protection against cell damage and aging process.
